# EUROPEAN PATENT SPECIFICATION

(11) **EP 2 245 654 B1**
(45) Date of publication and mention of the grant of the patent: **08.05.2013**
(21) Application number: 08871310.2
(22) Date of filing: 31.12.2008
(51) Int. Cl.: H01J 61/44, C09K 11/78, C09K 11/81, H01J 61/70

(54) **ENHANCED UV-EMITTING FLUORESCENT LAMP**
VERBESSERTE UV-EMITTIERENDE FLUORESZENZLAMPE
LAMPE FLUORESCENTE À ÉMISSION D'ULTRAVIOLETS AMÉLIORÉE

(30) Priority: 21.01.2008 US 17078
(43) Date of publication of application: 03.11.2010
(73) Proprietor: NARVA Lichtquellen GmbH & Co. KG, 09618 Brand-Erbisdorf (DE)
(72) Inventor: CAMIRE, Nathalie, J2C 4E6 Drummondville Québec (CA); DESBIENS, Nicolas, St-Germain-de-Grantham Québec J0C 1K0 (CA); DUTTA, Arunava, Winchester Massachusetts 01890 (US)
(74) Representative: Castell, Klaus
(86) International application number: PCT/US2008/088629
(87) International publication number: WO 2009/094100

(56) References cited:
- WO-A2-2007/038467
- DE-U1-202005 018 335

## Description

### Cross References to Related Applications

This application claims priority benefits of U.S. application Serial No. 12/017,078, filed January 21, 2008, which is a continuation-in-part of U.S. application Serial No. 11/525,942 filed 9/25/2006, which claims the benefit of U.S. provisional application Serial No. 60/596,513, filed 9/29/2005.

### Background of the Invention

Conventional fluorescent tanning lamps are basically low-pressure mercury discharge lamps that have a coating of at least one UV-emitting phosphor on the interior surface of the envelope. The typical geometry is a linear tubular shape though other shapes such as the spirals used in compact fluorescent lamps are also possible. The important lamp parameters for tanning purposes are generally 0h UVA, 0hTe and 100h UVA maintenance. The 0h UVA is the initial UVA flux produced by the lamp, 0h Te is the initial erythemal time and 100h UVA maintenance is the percentage of the initial UVA flux from the lamp that is available after 100h of lamp operation. The maximum exposure time (Te) is calculated according to the method prescribed by the U.S. Food and Drug Administration. *See, e.g.,* HHS Publication FDA 88-8234, "Quality Control Guide for Sunlamp Products," (March 1988). The initial erythemal time, 0h Te, is the value of Te calculated for the initial operation of the lamp after a brief period of stabilization.

Tanning lamps in the market today are designed exclusively for tanning which is not surprising. However, UV radiation is also able to help the human body produce vitamin D. It would therefore be advantageous to create a UV-emitting light source that would both tan and promote vitamin D synthesis in the human body. For example, this could benefit people who for a number of different reasons are unable to go out in the sunlight to promote vitamin D synthesis in the body or it might also be an attractive alternative for people who cannot process vitamin D enhanced food.
WO 2007/038467 A2 discloses a UV-emitting fluorescent lamp, comprising a sealed tubular envelope and at least one electrode for generating a discharge, the envelope containing an amount of mercury and having a phosphor coating on an interior surface. The phosphor coating comprising a mixture of a YPO₄:Ce phosphor, LaPO₄:Ce phosphor and SrB₄O₇:Eu phosphor, with three components in the blend ranging from 10wt.% to 25wt.% SrB₄O₇:Eu, 50wt.% to 70wt.% YPO₄:Ce and 10wt.% to 30wt.% LaPO₄:Ce with the sum of wt.% of the three components in the blend adding to 100%. It further relates to a lamp having a reflecting layer comprising aluminium oxide.

### Summary of the Invention

It is an object of the invention to obviate the disadvantages of the prior art.

It is a further object of the invention to provide a lamp that will perform adequately both as a tanning lamp and as a vitamin D enhancing lamp.

In accordance with one objection of the invention, there is provided a UV-emitting lamp containing a UV-emitting phosphor blend wherein the lamp when operating exhibits a vitamin D ratio of 1.5 to 2, a Hpi:Her ratio of 0.85 to 1, and a 0h Te of 30 to 40 minutes and the phosphor blend contains a SrB₄O₇:Eu phosphor, a LaPO₄:Ce phosphor and a YPO₄:Ce phosphor wherein the sum of the weight percentages of the phosphors in the blend is 100%.

In accordance with the invention, the phosphor blend comprises 25-27% SrB₄O₇:Eu, 23-26% LaPO₄:Ce, and 47-52% YPO₄:Ce by weight.

### Brief Description of the Drawings

Figure 1 shows the Vitamin D CIE 2006, Immediate Pigmentation and IEC Total Erythemal Effectiveness response functions.

Fig. 2 is a simplex centroid design for a three-component blend of SrB₄O₇:Eu, LaPO₄:Ce and YPO₄:Ce phosphors.

Fig. 3 is a plot the dependence of 0h UVA within the phosphor blend composition space illustrated in Fig. 2.

Fig. 4 is a plot of the dependence of 0h Te within the phosphor blend composition space illustrated in Fig. 2.

Fig. 5 is a plot of the dependence of the 100h UVA maintenance within the phosphor blend composition space illustrated in Fig. 2.

Fig. 6 is a plot of the dependence of the vitamin D ratio within the phosphor blend composition space illustrated in Fig. 2.

Fig. 7 is a plot of the dependence of the Hpi:Her ratio within the phosphor blend composition space illustrated in Fig. 2.

Fig. 8 shows the region of the phosphor blend composition space that is able to simultaneously satisfy a vitamin D ratio of 1.5 to 2, a Hpi:Her ratio of 0.85 to 1, and a 0h Te of 30 to 40 minutes.

Fig. 9 is an illustration of a longitudinal cross section of a reflector tanning lamp.

Fig. 10 is an illustration of a perpendicular cross section of a reflector tanning lamp.

### Detailed Description of the Invention

For a better understanding of the present invention, together with other and further objects, advantages and capabilities thereof, reference is made to the following disclosure and appended claims taken in conjunction with the above-described drawings.

The vitamin D enhancing ability of a lamp is determined by the vitamin D ratio which is defined as the ratio of the vitamin D CIE 2006 Flux (W/m²) to the Total IEC Erythemal Effective Irradiance (W/m²). The target vitamin D ratio that is desired is in the range of 1.5-2. For adequate tanning ability, this lamp must simultaneously have a suitable value for a second response called the Hpi:Her ratio. This is defined as a numerical factor (0.0025) times the ratio of the Immediate Pigmentation Flux (W/m²) to the Total IEC Erythemal Effective Irradiance (W/m²). The target Hpi:Her ratio is 0.85-1. Finally, this lamp must at the same time have a suitable value for 0h Te (the initial erythemal time) response which is desired to be in the range of 30-40 minutes.

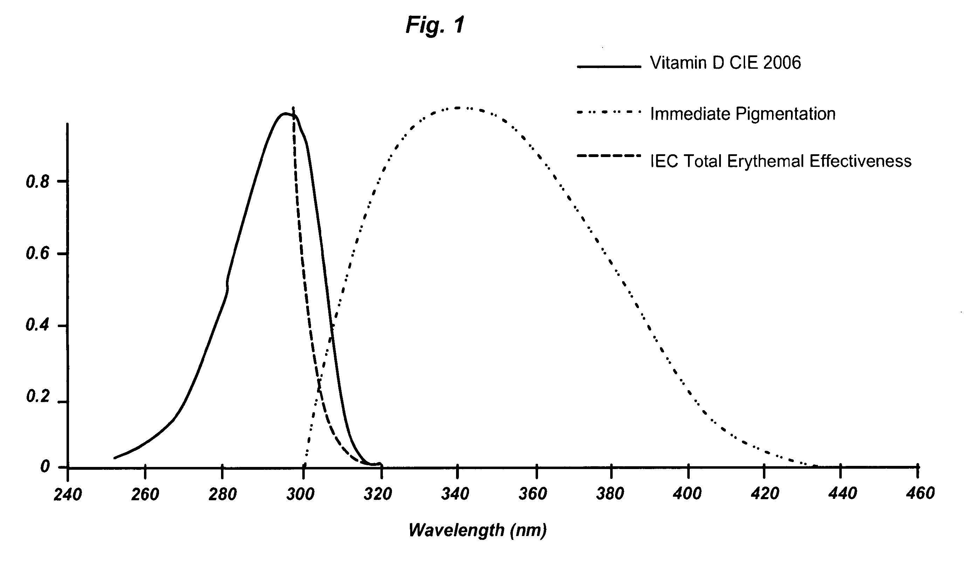

Figure 1 shows the three response functions of interest: Vitamin D CIE 2006, Immediate Pigmentation and IEC Total Erythemal Effectiveness. Each one shows the dependence of the particular response on wavelength. It is clear that each response depends quite differently on wavelength of the radiation emitted by the lamp. To obtain the flux for any response, the vitamin D CIE 2006 flux for example, the response function (in this case the vitamin D response function) has to be weighted by the lamp spectral power distribution (SPD). It also follows from the different response functions that while a lamp may have good tanning ability it may have a poor vitamin D ratio and vice versa.

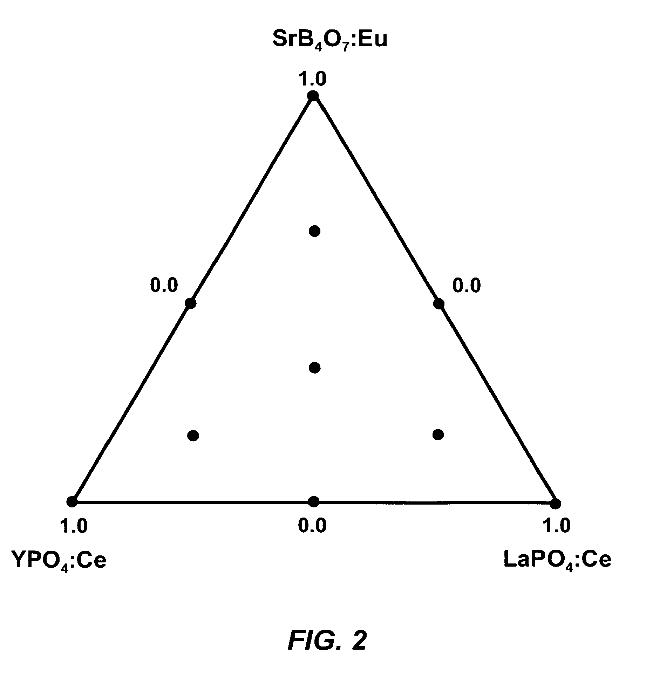

Three rare-earth-activated UV-emitting phosphors were selected for making tanning lamps. SrB₄O₇:Eu, LaPO₄:Ce and YPO₄:Ce. The SrB₄O₇:Eu phosphor has a peak emission at about 366 nm. The LaPO₄:Ce phosphor has a bimodal emission at about 316 nm and 338 nm. The YPO₄:Ce phosphor also has a bimodal emission at about 338 nm and 356 nm. A simplex centroid design was made to create ten different blends that have one or more of these phosphors. This design is shown in Figure 2.

In Figure 2, the three vertices of the triangle represent pure components, the three mid points on the sides of the triangle represent a two-component 50:50 blend of the phosphors at the end vertices, and the four points inside the triangle are three-component blends of the phosphors. The point located at the center of the triangle is the centroid or a blend with equal proportions of all three phosphors. The other three points represent blends having a 2/3, 1/6, 1/6 composition, where the component vertex closest to the point comprises the 2/3 fraction of the blend and the component vertices further away each comprise a 1/6 fraction. All of the blend proportions and percentages described herein are based on the weights of the individual phosphor components unless otherwise indicated.

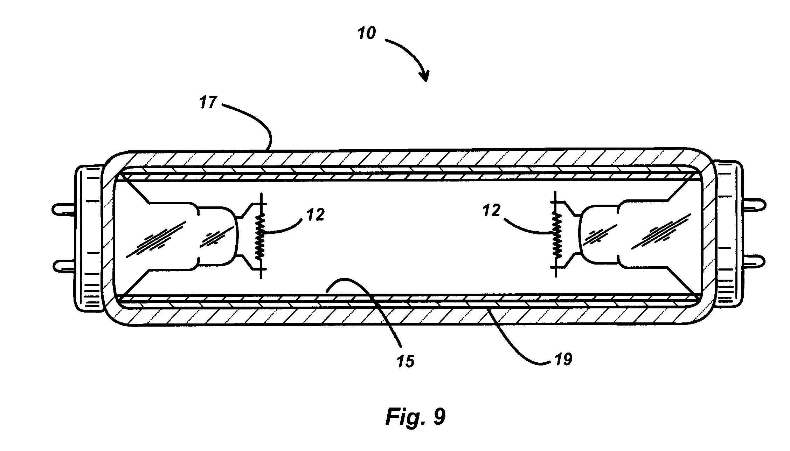

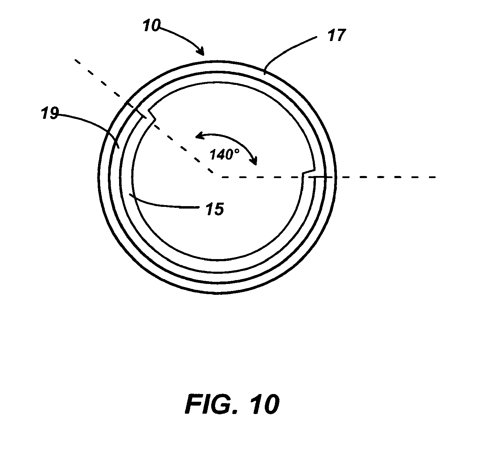

Reflector lamps (similar to that illustrated in Figures 9 and 10) were made that had been coated with each of the ten different phosphor blends. Ten lamps of each of the ten blends were photometered for 0h UVA, 0h Te and 100h UVA maintenance. In addition, the spectral power distributions for these lamps were measured and the response functions shown in Figure 1 used to determine the vitamin D ratio and Hpi:Her ratio for all ten phosphor blends.

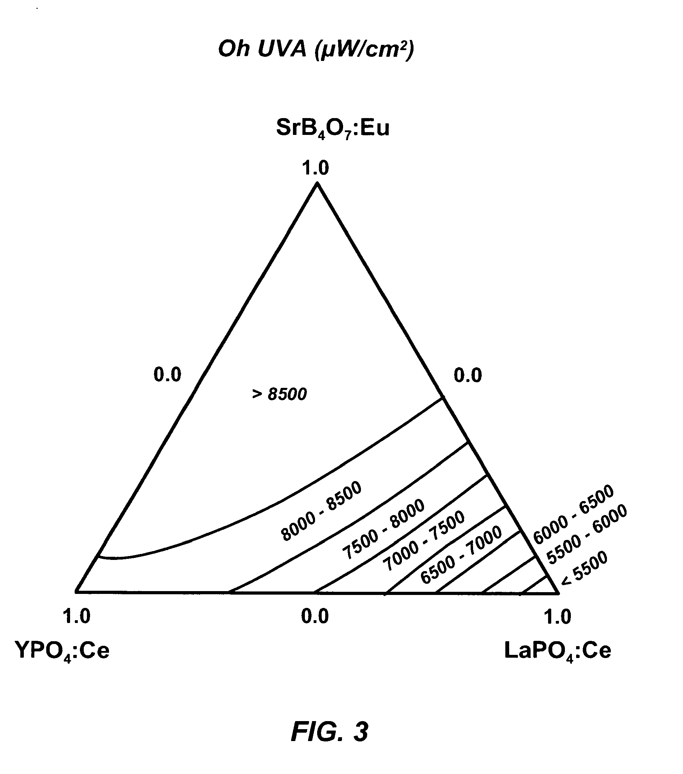

Figure 3 below shows the dependence of 0h UVA on the phosphor blend composition. It is seen that blend compositions rich in the SrB₄O₇:Eu phosphor result in higher 0h UVA while blend compositions rich in LaPO₄:Ce phosphor result in lower values of 0h UVA. It is desirable to have a value of 0h UVA that is > 8000 µW/cm².

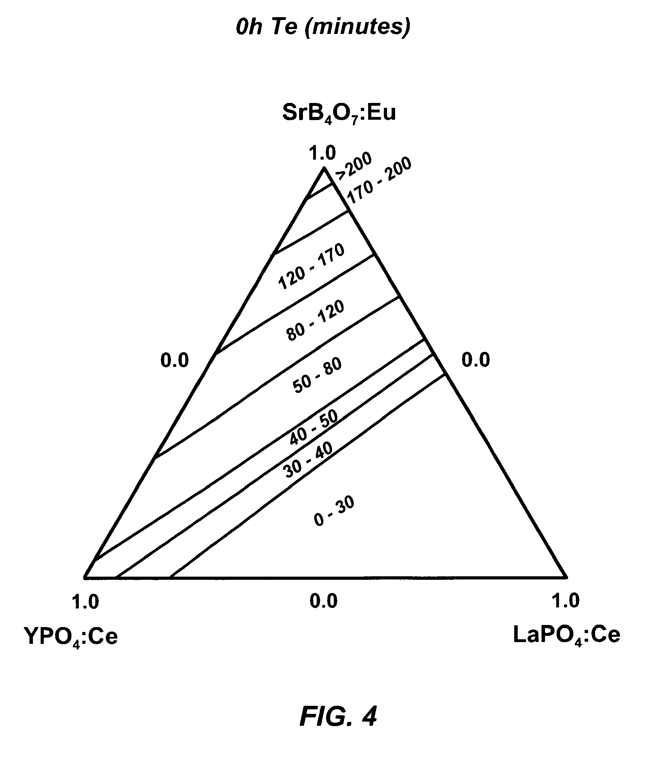

The dependence of 0h Te on phosphor blend composition is shown in Figure 4. It is clear from Figure 4 that higher levels of LaPO₄:Ce in the blend results in faster (shorter) 0h Te for the lamp while increasing the percentage of SrB₄O₇:Eu in the blend results in a slower (longer) 0h Te. The phosphor blend region that would give a lamp 0h Te in the preferred 30-40 minute range is identified in Figure 4.

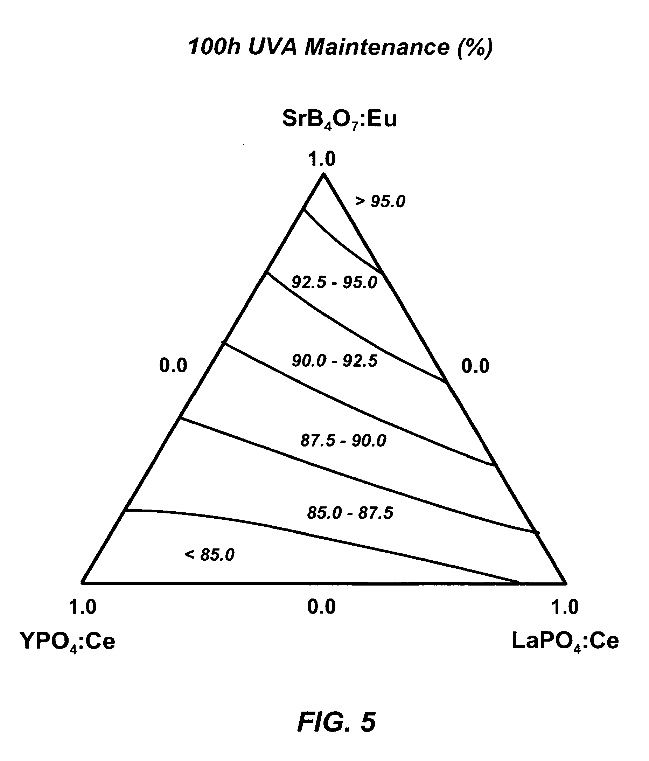

The 100h UVA maintenance of the lamps is shown in Figure 5. While the maintenance is generally good, >85%, for any composition involving these three phosphors, it is seen that increasing the level of the SrB₄O₇:Eu phosphor in the blend relative to the phosphate phosphors increases the 100h UVA maintenance further.

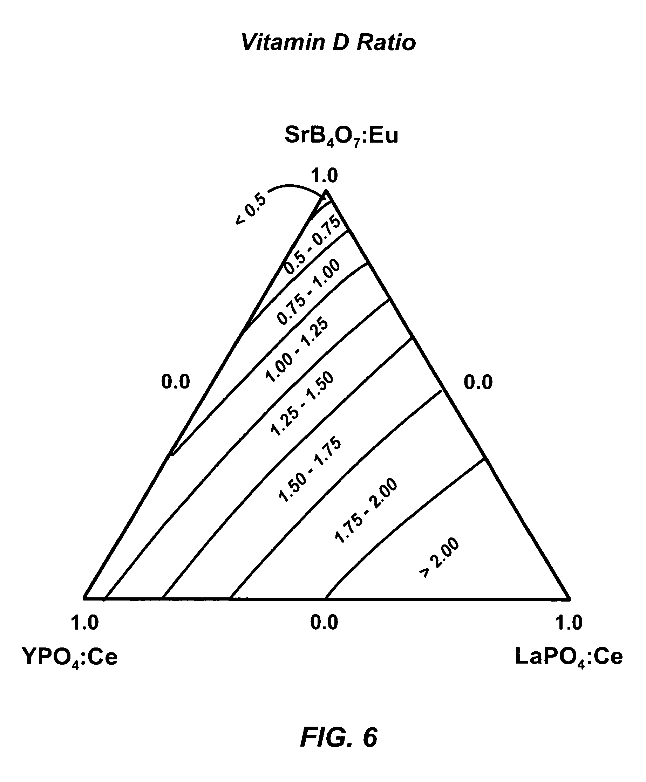

The vitamin D ratio for all ten different phosphor blends is determined from the response functions shown in Figure 1 and the lamp SPD for each of the ten blends. The dependence of the vitamin D ratio on the phosphor blend composition is shown in Figure 6. It is observed from Figure 6 that the higher the level of LaPO₄:Ce phosphor in the blend the higher the vitamin D ratio. In other words moving towards the LaPO₄:Ce vertex in the triangle increases the vitamin D ratio whereas moving towards the SrB₄O₇:Eu vertex lowers the vitamin D ratio. The particular phosphor blend space that allows the desired vitamin D ratio of >1.5 is also shown in Figure 6.

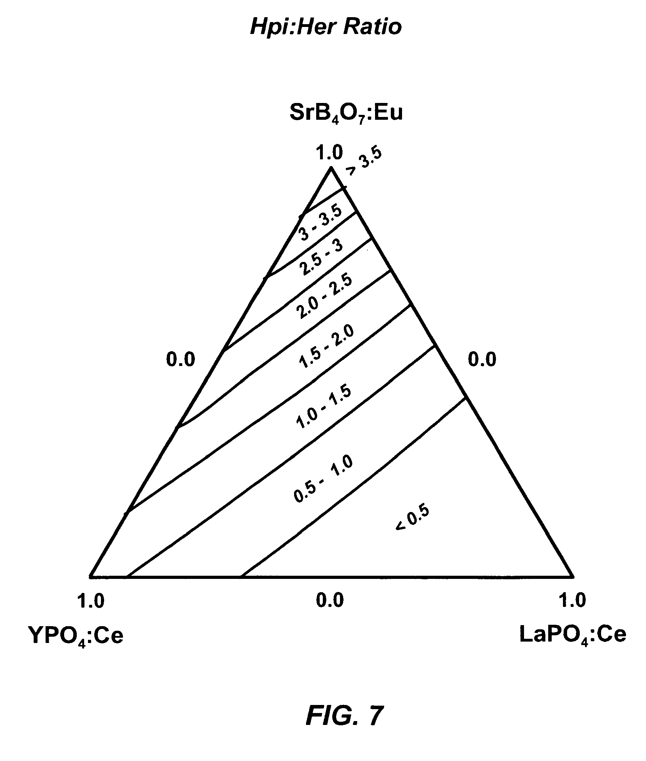

The Hpi:Her ratio was also determined from the response functions shown in Figure 1 and the SPD of the lamps for all ten different phosphor blends. The dependence of the Hpi:Her ratio on phosphor blend composition is shown in Figure 7. As described above, the Hpi:Her ratio is defined as a numerical factor (0.0025) times the ratio of the Immediate Pigmentation Flux (W/m²) to the Total IEC Erythemal Effective Irradiance (W/m²). The target Hpi:Her ratio is 0.85-1 for adequate tanning ability.

It is clear from Figure 7 that higher values of Hpi:Her are obtained by moving towards the SrB₄O₇:Eu vertex. Yet Figure 6 indicates that for higher values of the vitamin D ratio one must travel in the other direction towards the LaPO₄:Ce vertex. Also for suitable values of 0h Te, it would be preferred from Figure 4 not to have high levels of SrB₄O₇:Eu phosphor in the blend.

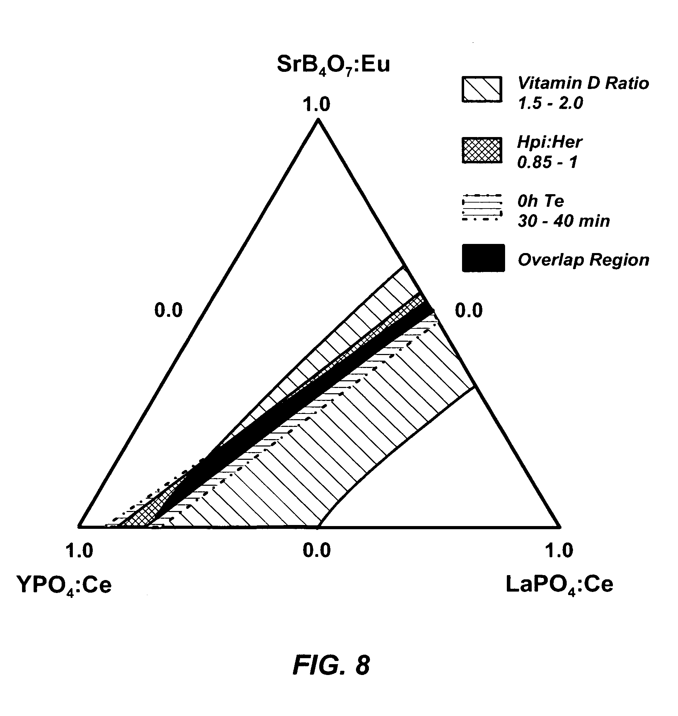

Using the data shown in Figures 4, 6, and 7 there is a small region of the phosphor blend composition space that is able to satisfy all three desired criteria simultaneously: a vitamin D ratio of 1.5 to 2, a Hpi:Her ratio of 0.85 to 1, and a 0h Te of 30 to 40 minutes. This overlap region is shown in Figure 8. Preferably, the phosphor blend comprises 25-27% SrB₄O₇:Eu, 23-26% LaPO₄:Ce, and 47-52% YPO₄:Ce by weight.

A blend was selected from this narrow region and used to make reflector lamps. The blend was 25.3% SrB₄O₇:Eu, 25.4% LaPO₄:Ce and 49.3% YPO₄:Ce. The properties of the finished lamps are shown below in Table 1:

**Table 1**

| | |
|---|---|
| Vitamin D ratio | 1.7 |
| Hpi:Her ratio | 0.85 |
| 0h Te | 34 minutes |
| 0h UVA | 8170 µW/cm² |
| 100h UV maintenance | 87% |

Deviation from this particular blend by more than 2 percentage points in the direction of increasing the amount SrB₄O₇:Eu phosphor will cause the vitamin D ratio to drop below the desirable target level. The result will be a lamp that will tan but will not be effective for vitamin D production.

An illustration of a typical reflector tanning lamp is shown in Figures 9 and 10. Figure 9 illustrates a longitudinal cross section through the tubular lamp along its central axis. Figure 10 illustrates a cross section perpendicular to the central axis of the lamp. The lamp 10 has a hermetically sealed UV transmissive, glass envelope 17. The interior of the envelope 17 is filled with an inert gas such as argon, neon, krypton or a mixture thereof, and a small quantity of mercury, at least enough to provide a low vapor pressure during operation. An electrical discharge is generated between electrodes 12 to excite the mercury vapor to generate ultraviolet radiation. A coating of a UV reflective material 19, e.g., aluminum oxide (alumina), is coated on the interior surface of the envelope 17 and a phosphor layer 15 is applied over the reflective layer 19. For a lamp according to this invention, the phosphor layer 15 contain the blend of the three phosphors, SrB₄O₇:Eu, LaPO₄:Ce and YPO₄:Ce. While the phosphor layer 15 covers the entire bulb circumference, a typical coverage angle for the reflector layer varies from 180° to 240° of the circumference. A reflector layer that covers 220° of the circumference is shown in Figure 10. The primary role of the reflector material is to reflect the UV radiation emitted by the phosphor layer back towards the front of the lamp from where it escapes through the region of the bulb that does not have any UV reflective material on the glass.

## Claims

1. A UV-emitting fluorescent lamp, comprising a sealed tubular envelope and at least one electrode for generating a discharge, the envelope containing an amount of mercury and having a phosphor layer on an interior surface; the phosphor layer containing a phosphor blend comprising a mixture of a SrB₄O₇:Eu phosphor, a LaPO₄:Ce phosphor and a YPO₄:Ce phosphor wherein the sum of the weight percentages of the phosphors in the blend is 100%; and wherein the phosphor blend comprises 25-27% by weight SrB₄O₇:Eu, 23-26% by weight LaPO₄:Ce, and 47-52% by weight YPO₄:Ce.

2. The lamp of claim 1 wherein the phosphor blend comprises 25.3% by weight SrB₄O₇:Eu, 25.4% by weight LaPO₄:Ce, and 49.3% by weight YPO₄:Ce.

3. The lamp of claim 1 wherein the lamp has a 0 h UVA of > 8000 µW/cm².

4. The lamp of claim 1 wherein the lamp has a 100h UVA maintenance of >85%.

5. The lamp of claim 1 wherein the UV-reflective layer disposed between the phosphor layer and the envelope, the UV-reflective layer extending partially around the circumference of the envelope and comprising aluminium oxide.

6. The lamp of claim 5 wherein the coverage angle of the UV-reflective layer is from 180° to 240° of the circumference.

7. The lamp of claim 5 wherein the coverage angle of the UV-reflective layer is 220° of the circumference.

## Patentansprüche

1. UV-emittierende Fluoreszenzlampe, umfassend einen abgedichteten röhrenförmigen Kolben und mindestens eine Elektrode zum Erzeugen einer Entladung, wobei der Kolben eine Menge an Quecksilber enthält und eine Phosphorschicht an einer Innenfläche aufweist; wobei die Phosphorschicht eine Phosphormischung enthält, die ein Gemisch aus einem SrB₄O₇:Eu-Phosphor, einem LaPO₄:Ce-Phosphor und einen YPO₄:Ce-Phosphor umfasst, wobei die Summe der Gewichtsprozente der Phosphore in der Mischung 100% ist; und wobei die Phosphormischung 25-27 Gewichtsprozent SrB₄O₇:Eu, 23-26 Gewichtsprozent LaPO₄:Ce und 47-52 Gewichtsprozent YPO₄:Ce umfasst.

2. Lampe nach Anspruch 1, wobei die Mischung 25,3 Gewichtsprozent SrB₄O₇:Eu, 25,4 Gewichtsprozent LaPO₄:Ce und 49,3 Gewichtsprozent YPO₄:Ce umfasst.

3. Lampe nach Anspruch 1, wobei die Lampe einen 0 h UVA-Wert von > 8000 µW/cm² hat.

4. Lampe nach Anspruch 1, wobei die Lampe einen 100 h UVA-Erhaltungsfaktor von > 85% hat.

5. Lampe nach Anspruch 1, wobei die UV-reflektierende Schicht zwischen der Phosphorschicht und dem Kolben angeordnet ist, wobei sich die UV-reflektierende Schicht teilweise um den Umfang des Kolbens erstreckt und Aluminiumoxid umfasst.

6. Lampe nach Anspruch 5, wobei der Erfassungswinkel der UV-reflektierenden Schicht 180° bis 240° des Umfangs ist.

7. Lampe nach Anspruch 5, wobei der Erfassungswinkel der UV-reflektierenden Schicht 220° des Umfangs ist.

## Revendications

1. Lampe fluorescente à émission d'UV, comprenant une enveloppe tubulaire étanche et au moins une électrode pour générer une décharge, l'enveloppe contenant un volume de mercure et ayant une couche de phosphore sur une surface intérieure ; la couche de phosphore contenant un mélange de phosphore comprenant un mélange d'un phosphore SrB₄O₇:Eu, un phosphore LaPO₄:Ce et un phosphore YPO₄:Ce où la somme des pourcentages de poids des phosphores dans le mélange est de 100%; et où le mélange de phosphore comprend 25-27% en poids de SrB₄O₇:Eu, 23-26% en poids de LaPO₄:Ce, et 47-52% en poids de YPO₄:Ce.

2. Lampe de la revendication 1 où le mélange de phosphore comprend 25,3% en poids de SrB₄O₇:Eu, 25,4% en poids de LaPO₄:Ce, et 49,3% en poids de YPO₄:Ce.

3. Lampe de la revendication 1 où la lampe a un 0 h UVA de > 8000 µW/cm².

4. Lampe de la revendication 1 où la lampe a une maintenance 100 h UVA de > 85%.

5. Lampe de la revendication 1 où la couche de réflexion d'UV disposée entre la couche de phosphore et l'enveloppe, la couche de réflexion d'UV se prolongeant partiellement autour de la circonférence de l'enveloppe et comprenant de l'oxyde d'aluminium.

6. Lampe de la revendication 5 où l'angle de couverture de la couche de réflexion d'UV va de 180° à 240° de la circonférence.

7. Lampe de la revendication 5 où l'angle de couverture de la couche de réflexion d'UV est 220° de la circonférence.
